# EUROPEAN PATENT SPECIFICATION

(11) **EP 1 237 575 B1**
(45) Date of publication and mention of the grant of the patent: **06.08.2008**
(21) Application number: 00986377.0
(22) Date of filing: 14.12.2000
(51) Int. Cl.: A61K 39/395, A61K 38/17, A61P 37/06, A61P 29/00

(54) **TNF-ALPHA ANTAGONIST AND LFA-1 ANTAGONIST FOR TREATING RHEUMATOID ARTHRITIS**
LFA-1 ANTAGONISTEN UND TNF-ALPHA ANTAGONISTEN ZUR BEHANDLUNG VON RHEUMATOIDEN ARTHRITIS
ANTAGONISTE DE TNF-ALPHA ET ANTAGONISTE DE LFA-1 DESTINES A TRAITER L' ARTHRITE RHUMATOïDE

(30) Priority: 14.12.1999 US 170696 P
(43) Date of publication of application: 11.09.2002
(73) Proprietor: GENENTECH, INC., South San Francisco, CA 94080-4990 (US)
(72) Inventor: LEE, Wyne, Pun, Millbrae, CA 94030-2320 (US); TUMAS, Daniel, B., Orinda, CA 94563 (US)
(74) Representative: Kiddle, Simon John
(86) International application number: PCT/US2000/033875
(87) International publication number: WO 2001/043773

(56) References cited:
- WO-A-98/22137
- WO-A-98/23761
- MORELAND L W ET AL: "LONGTERM TREATMENT OF DMARD FAILING RHEUMATOID ARTHRITIS PATIENTS WITH TNF RECEPTOR P75 FC FUSION PROTEIN (TNF;FC ENBREL)" JOURNAL OF INVESTIGATIVE MEDICINE,AMERICAN FEDERATION FOR CLINICAL RESEARCH,,US, vol. 46, 1998, page 229A XP000972312 ISSN: 1081-5589
- GAO J -X ET AL: "Mac-1 (CD11b/CD18) is the predominant beta-2 (CD18) integrin mediating human neutrophil migration through synovial and dermal fibroblast barriers." IMMUNOLOGY, vol. 88, no. 3, 1996, pages 463-470, XP002167334 ISSN: 0019-2805
- LOVELL D I ET AL: "SAFETY AND EFFICACY OF TUMOR NECROSIS FACTOR RECEPTOR P75 FC FUSION PROTEIN (TNFR-FC, ENBREL)IN POLYARTICULAR COURSE JUVENILE RHEUMATOID ARTHRITIS" ARTHRITIS AND RHEUMATISM,LIPPINCOTT, PHILADELPHIA,US, vol. 41, no. 9, SUPPL, 8 November 1998 (1998-11-08), page S130 XP000960458 ISSN: 0004-3591
- SPENCER-GREEN G: "ETANERCEPT (ENBREL): UPDATE ON THERAPEUTIC USE" ANNALS OF THE RHEUMATIC DISEASES,BRITISH MEDICAL ASSOCIATION, LONDON,GB, vol. 59, no. SUPPL. 01, November 2000 (2000-11), pages 146-149, XP000965592 ISSN: 0003-4967

## Description

### Field of the Invention

The invention relates to treatment of arthritis with an LFA-I antagonist and a TNF-α antagonist, as defined in the claims.

### Backeround of the Invention

TNF is a naturally occurring cytokine that is involved in normal inflammatory and immune responses. It plays an important role in the inflammatory processes of rheumatoid arthritis (RA), polyarticular-course juvenile rheumatoid arthritis (JRA), and the resulting joint pathology. Elevated levels of TNF are found in the synovial fluid of RA patients. Two distinct receptors for TNF (TNFRs), a 55 kilodalton protein (p55) and a 75 kilodalton protein (p75), exist naturally as monomeric molecules on cell surfaces and in soluble forms. Biological activity of TNF is dependent upon binding to either cell surface TNFR. The p55 receptor (also termed TNF-R55. TNF-RI, or TNFR,beta) is a 55 kd glycoprotein shown to transduce signals resulting in cytotoxic, anti-viral, and proliferative activities of TNF-α. The p75 receptor (also termed TNF-R75, TNF-RII, or TNFR-α) is a 75 kDa glycoprotein that has also been shown to transduce cytotoxic and proliferative signals as well as signals resulting in the secretion of GM-GSF.

Monocytes and macrophages secrete cytokines known as tumor necrosis factor-alpha (TNF-α) and tumor necrosis factor-beta (TNF-β; lymphotoxin) in response to endotoxin or other stimuli. TNF-α is a soluble homotrimer of 17 kD protein subunits (Smith, et al., J. Biol. Chem. 262:6951-6954 (1987)). A membrane-bound 26 kD precursor form of TNF also exists (Kriegler, et al., Cell 53:45-53 (1988)). For reviews of TNF. see Beutler, et al., Nature 320:584 (1986), Old. Science 230:630 (1986), and Le, et al., Lab. Invest. 56:234. Cells other than monocytes or macrophages also make TNF-α. For example, human non-monocytic tumor cell lines produce TNF (Rubin, et al., J. Exp. Med. 164:1350 (1986): Spriggs, et al., Proc. Nail. Acad. Sci. USA 84:6563 (1987)). CD4⁻ and CD8⁻ peripheral blood T lymphocytes and some cultured T and B cell lines (Cuturi, et al., J. Exp. Med. 165:1581 (1987); Sung, et al., J. Exp. Med. 168:1539 (1988)) also produce TNF-α.

TNF causes pro-inflammatory actions which result in tissue injury, such as inducing procoagulant activity on vascular endothelial cells (Pober, et al., J. lmmunol. 136:1680 (1986)), increasing the adherence of neutrophils and lymphocytes (Pober, et al., J. Immunol. 138:3319 (1987)), and stimulating the release of platelet activating factor from macrophages, neutrophils and vascular endothelial cells (Camussi, et al., J. Exp. Med. 166:1390 (1987)). TNF is also associated with infections (Cerami, et al., lmmunol. Today 9:28 (1988)), immune disorders, neoplastic pathologies (Oliff, et al., Cell 50:555 (1987)), autoimmune pathologies and graft-versus host pathologies (Piguet, et al.. J. Exp. Med. 166:1280 (1987)).

TNF also plays a central role in gram-negative sepsis and endotoxic shock (Michie, et al., Br. J. Surg. 76:670-671 (1989): Debets, et al., Second Vienna Shock Forum, p.463-466 (1989): Simpson, et al., Crit. Care Clin. 5:27-47 (1989): Waage, et al., Lancet 1:355-357 (1987): Hammerle, et al., Second Vienna Shock Forum p. 715-718 (1989); Debets, et al., Crit. Care Med. 17:489-497 (1989); Calandra, et al., J. Infect. Dis. 161:982-987 (1990); Revhaug, et al., Arch. Surg. 123:162-170 (1988)), including fever, malaise, anorexia, and cachexia.

Polyclonal murine antibodies to TNF are disclosed by Cerami et al. (EPO Patent Publication 0212489, Mar. 4, 1987). Such antibodies were said to be useful in diagnostic immunoassays and in therapy of shock in bacterial infections. Rubin et al, (EPO Patent Publication 0218868. Apr. 22, 1987) discloses murine monoclonal antibodies to human TNF, the hybridomas secreting such antibodies, methods of producing such murine antibodies, and the use of such murine antibodies in immunoassay of TNF.

Yone et al. (EPO Patent Publication 0288088, Oct. 26. 1988) disclose anti-TNF murine antibodies, including mAbs, and their utility in immunoassay diagnosis of pathologies, in particular Kawasaki's pathology and bacterial infection. The body fluids of patients with Kawasaki's pathology (infantile acute febrile mucocutaneous lymph node syndrome; Kawasaki, Allergy 16:178 (1967); Kawasaki. Shonica (Pediatrics) 26:935 (1985)) were said to contain elevated TNF levels which were related to progress of the pathology (Yone et al., infra).

Other investigators have described rodent or murine mAbs specific for recombinant human TNF which had neutralizing activity in vitro (Liang, et al., (Biochem. Biophys. Res. Comm. 137:847-854 (1986): Meager, et al., Hybridoma 6:305-311 (1987): Fendly et al., Hybridoma 6:359-369 (1987); Bringman, et al., Hybridoma 6:489-507 (1987); Hirai, et al., J. Immunol. Meth. 96:57-62 (1987); Moller, et al., (Cytokine 2:162-169 (1990)). Some of these mAbs were used to map epitopes of human TNF and develop enzyme immunoassays (Fendly et al.. infra; Hirai et al., infra; Moller et al., infra) and to assist in the purification of recombinant TNF (Bringman et al., infra). However, these studies do not provide a basis for producing TNF neutralizing antibodies that can be used for in vivo diagnostic or therapeutic uses in humans, due to immunogenicity, lack of specificity and/or pharmaceutical suitability.

Neutralizing antisera or mAbs to TNF have been shown in mammals other than man to abrogate adverse physiological changes and prevent death after lethal challenge in experimental endotoxemia and bacteremia. This effect has been demonstrated, e.g.. in rodent lethality assays and in primate pathology model systems (Mathison, et al.. J. Clin. Invest. 81:1935-1937 (1988): Beutler, et al.. Science 229:869-871 (1985); Tracey, et al.. Nature 330:662-664 (1987); Shimamoto, et al., Immunol. Lett, 17:311-318 (1988); Silva, et al., J. Infect. Dis. 162:421-427 (1990); Opal, et al., J. Infect. Dis, 161:1148-1152 (1990); Hinshaw, et al., Circ. Shock 30:279-292 (1990)).

Putative receptor binding loci of hTNF has been disclosed by Eck and Sprang (J. Biol. Chem. 264(29). 17595-17605 (1989), who identified the receptor binding loci of TNF-α as consisting of amino acids 11-13.37-42, 49-57 and 155-57.

PCT publication W091/02078 (1991) discloses TNF ligands which can bind to monoclonal antibodies having certain epitopes.

To date, experience with anti-TNF murine mAb therapy in humans has been limited. In a phase 1 study, fourteen patients with severe septic shock were administered a murine anti-TNF mAb in a single dose from 0.4-10 mg/kg (Exley, A. R. et al., Lancet 335:1275-1277 (1990)). However, seven of the fourteen patients developed a human anti-murine antibody response to the treatment, which treatment suffers from the known problems due to immunogenicity from the use of murine heavy and light chain portions of the antibody. Such immunogenicity causes decreased effectiveness of continued administration and can render treatment ineffective, in patients undergoing diagnostic or therapeutic administration of murine anti-TNF antibodies.

Administration of murine TNF mAb to patients suffering from severe graft versus host pathology has also been reported (Herve, et al., Lymphoma Res. 9:591 (1990)).

ENBREL (etanercept) is a dimeric fusion protein consisting of the extracellular ligand-binding portion of the human 75 kilodalton (p75) tumor necrosis factor receptor (TNFR) linked to the Fc portion of human IgG1. The Fc component of etanercept contains the CH2 domain, the CH3 domain and hinge region, but not the CH I domain of IgG1. Etanercept binds specifically to tumor necrosis factor (TNF) and blocks its interaction with cell surface TNF receptors. It inhibits the activity of TNF and has been shown to affect several animal models of inflammation, including murine collagen-induced arthritis. The main treatment for RA has been with methotrexate. Etanercept is currently being used in the treatment of arthritis: the therapy is described in e.g., Moreland et al., 1999. Ann. Intern. Med. 130:478-486.

Aderka, et al., Isrl. J. Med. Sci. 28:126-130 (1992) discloses soluble forms of TNF receptors (sTNF-Rs) which specifically bind TNF and thus can compete with cell surface TNF receptors to bind TNF (Seckinger, et al.. J. Exp. Med. 167:1511-1516 (1988); Engelmann, et al.. J. Biol. Chem. 264:11974-11980 (1989)). The cloning and expression of human 55 kd TNF receptor and soluble forms of the receptor have been described (Loetscher, et al., Apr. 20, 1990, Cell 61:351-359; Schall et al., Apr. 20, 1990, Cell 61:361-370: Nophar, et al., EMBO J. 9(10):3269-3278 (1990). Engelmann, et al., J. Biol. Chem. 265(3):1531-1536 (1990), discloses TNF-binding proteins. EP 0 433 900 A I discloses TNF binding protein I (TBP-1), derivatives and analogs thereof, expression of a DNA encoding the entire human type 1 TNF receptor, or a soluble domain thereof. WO 92/13095 discloses methods for treating tumor necrosis factor mediated diseases by administration of a therapeutically effective amount of a TNF inhibitor selected from a 30 kDa TNF inhibitor and a 40 kDa TNF inhibitor.

EP 0 526 905 discloses multimers of the soluble forms of TNF receptors, which include portions of the hp55 TNF-receptor, produced by either chemical or recombinant methods which are useful for protecting mammals from the deleterious effects of TNF. WO 92/07076 discloses modified human TNF-α receptor which consists of the first three cysteine-rich subdomains but lacks the fourth Cysteine-rich subdomain of the extracellular binding domain of the 55 kDa or 75 kDa TNF receptor for human TNF-α, or an amino acid sequence having a horology of 90% or more with the TNF receptor sequences. EP 0 412 486 A I discloses antibodies to TNF binding protein I (TBP-1), and fragments thereof, which can be used as diagnostic assays or pharmaceutical agents, either inhibiting or mimicking the effects of TNF on cells. EP 0 398 327 A1 discloses TNF binding protein (TBP) isolated and purified having inhibitory activity on the cytotoxic effect of TNF. as well as TNF binding protein 11 and monoclonal antibodies thereto. EP 0 308 378 A2 discloses TNF inhibitory protein and functional derivatives used to antagonize the deleterious effects of TNF.

LFA-I (consisting of CD11a and CD18 subunits) interaction with ICAM is necessary for T-cell killing. T-helper and B-cell responses, natural killing, and antibody-dependent cytotoxicity. In addition. LFA-l'ICAM interactions are involved in adherence of leukocytes to endothelial cells, fibroblasts, and epithelial cells, facilitating the migration of leukocytes from the vasculature to the sites of inflammation (Collins. T., 1995. Science and Medicine, 28-37; Dustin, ML. et al., 1991, Annual Rev Immunology, 9:27-66).

Using antibodies that interfere with LFA-1/ICAM interactions decreases or inhibits the inflammatory process by blocking the activation of T-cells and/or the extravasation of leukocytes. *In vitro,* monoclonal antibodies against LFA-1 or its ligands have inhibited T-cell activation (Kuypers, T. and Roos. D.. 1989. Research in Immunology, 140:461-86; Springer, TA. 1987. Annual Rev Immunology, 5:223-52). T-cell dependent B-cell proliferation (Fischer, A. et al., 1986, J Immunol, 136:3198-203), target cell lysis (Krensky, A. et al., 1983, J Immunol, 131:6711-6), and adhesion of T-cells to vascular endothelium (Dustin, ML. et al., 1988. Journal of Cell Biology, 107:321-31). The use of an anti-CD11a antibody to treat psoriasis has been described in WO 0056363. In mice, anti-CD11a antibodies have induced tolerance to protein antigens (Benjamin. R. et al. 1988, European Journal of immunology, 18:1079-88: Tanaka, Y. et al., 1995, European Journal of Immunology, 25:1555-8), delayed the onset and reduced the severity of experimental autoimmune encephalomyelitis (Gordon, EJ et al., 1995, Journal of Neuroimmunology, 62:153-60), inhibited lupus-associated autoantibody production, and prolonged survival of several types of tissue grafts (Cavazzana-Calco MS, Sarnacki S. Haddad E. et al.. Transplantation 1995;59(11):1576-82; Nakakura EK. McCabe SM. Zheng B. Shorthouse RA. et al., Transplantation 1993;55(2):412-7; Connolly MK, Kitchens EA, Chan B, et al, Clinical Immunology and Immunopathology 1994:72(2):198-203; He Y, Mellon J, Apte R. Niederkom J., Investigative Ophthalmology and Visual Science 1994:35(8):3218-35: Isobe M. Yagita H. Okumura K. Ihara A.. Science 1992;255:1125-7; Kato Y, Yamataka A. Yagita H, et al., Ann Surg 1996:223(1):94-100; Nishihara M. Gotoh M. Fukuzaki T. et al.. Transplantation Proceedings 1995:27(1):372; Talento A, Nguyen M, Blake T, et al, Transplantation 1993:55(2):418-22; van Dijken PJ, Ghayur T. Mauch P. et al., Transplantation 1990:49(5):882-6). In human clinical studies, murine anti-CD11a monoclonal antibodies have been shown to help prevent graft failure following bone marrow transplantation (Cavazzana-Calco MS. Bordigoni P, Michel G, et al.. British Journal of Haematology 1996;93:131-8; Fischer A, Friedrich W, Fasth A.. Blood 1991;77(2):249-56; Stoppa AM, Maraninchi D, Blaise D, Viens P. et al., Transplant International 1991;4:3-7) and renal transplantation (Hourmant M, Le Mauff B, Le Meur Y, et al., Transplantation 1994;58(3):377-80; Hourmant M, Bedrossian J. Durand D, et al., Transplantation 1996;62(11):1565-70; Le Mauff B, Hourmant M, Rougier JP, et al., Transplantation 1991;52(2):291-6).

In rheumatoid arthritis, the main presenting symptoms are pain, stiffness, swelling, and loss of function (Bennett J C. The etiology of rheumatoid arthritis. In Textbook of Rheumatology (Kelley W N, Harris E D. Ruddy S, Sledge C B, eds.) W B Saunders. Philadelphia pp 879-886, 1985). The multitude of drugs used in controlling such symptoms seems largely to reflect the fact that none is ideal. None of the treatments clearly stop progression of joint destruction (Harris E D. Rheumatoid Arthritis: The clinical spectrum. In Textbook of Rheumatology (Kelley, et al., eds.) W B Saunders, Philadelphia pp 915-990, 1985).

TNF-α is of major importance in the pathogenesis of rheumatoid arthritis. TNF-α is present in rheumatoid arthritis joint tissues and synovial fluid at the protein and mRNA level (Buchan G. et al., Clin. Exp. Immunol 73: 449-455, 1988), indicating local synthesis.

The normal functional capacity of the joint is diminished in OA or rheumatoid arthritis. The suboptimal functional capacity of the joint cartilage in OA or RA thus predisposes the joint to damage insult including the normal level of mechanical or physical insult applied to the joint during activity. The joint cartilage in OA or RA is also less optimally able to undergo normal repair when damaged. Damage to the joint in OA and RA is thus often progressive and damaged hyaline joint cartilage can be replaced by sub-optimal fibrocartilage. Fibrocartilage has significant physical and biochemical differences than that of normal hyaline or articular cartilage in a normal joint and does not optimally have the same functional capacity.

The degradation associated with osteoarthritis usually initially appears as fraying and fibrillation of the surface. Loss of proteoglycan from the matrix also occurs. As the surface fibrillation progresses, the defects penetrate deeper into the cartilage and cartilage is lost. The subchondral bone thickens, is slowly exposed, and may appear polished. Bony nodules or osteophytes also often form at the periphery of the cartilage surface and occasionally grow over the adjacent eroded areas. If the surface of these bony outgrowths is permeated, vacular outgrowth may occur and cause the formation of tissue plugs containing fibrocartilage.

The use of peptide growth factors has also been examined to promote repair of damaged cartilage. Peptide growth factors are very significant regulators of cartilage growth and cell behavior (*i.e.,* differentiation, migration, division, or matrix synthesis or breakdown) [F. S. Chen et al., Am J. Orthop. 26: 396-406 (1997)].

Growth factors that have been previously proposed to stimulate cartilage repair include insulin-like growth factor (IGF-1), [Osborn, J. Orthop. Res. 7: 35-42 (1989); Florini & Roberts, J. Gerontol. 35: 23-30 (1980)]; basic fibroblast growth factor (bFGF), [Toolan et al., J. Biome. Mat. Res. 41: 244-50 (1998): Sah et al., Arch. Biochem. Biophys. 308: 137-47 (1994)]: bone morphogenetic protein (BMP) [Sato & Urist, Clin. Orthop. Relat. Res. 183: 180-87 (1984); Chin et al., Arthritis Rheum. 34: 314-24 (1991) and transforming growth factor beta (TGF-β) [Hill & Logan. Prog. Growth Fac. Res. 4: 45-68 (1992): Gueme et al., J. Cell Physiol. 158: 476-84 (1994); Van der Kraan et al., Ann. Rheum. Dis. 51: 643-47 (1992)]. Insulin has further been proposed to increase cartilage synthesis, insofar as cultured osteoarthritic cartilage explants treated with insulin and tritiated thymidine and [³⁵S]-sulfate showed incorporation of the latter in a general synthetic response. J. Posever et al., J. Orthopaedic Res. 13: 832-827 (1995). Other methods of stimulating cartilage repair include the antagonisation of molecules which are associated with or aggravate cartilage destruction and use, for example, IL-1α and nitric oxide.

The great majority of people with RA have a genetic susceptibility associated with increased activation of class 11 major histocompatibility complex molecules on monocytes and macrophages. The genetic predisposition to RA is further supported by the prevalence of the highly conserved leukocyte antigen DR subtype Dw4, Dw14 and Dw15 in human patients with very severe disease. The activated monocytes and macrophages, in interacting with the appropriate T cells stimulate a cascade or immune events which results in further activation of more monocytes and macrophages. T cells. B cells and endothelial cells. This activation increases the synthesis of adhesion molecules, resulting in attracting even more mononuclear cells and polymorphonuclear cells to the inflamed joint. This influx further results in the secretion of additional chemotactic cytokines, causing the invasion of even more inflammatory cells to the synovium and synovial fluid surrounding the joint.

It is generally believed, that many different arthriogenic stimuli activate the immune response in the immunogenetically susceptible host in RA. Both exogenous infectious agents (Ebstein-Barr Virus. Rubella virus. Cytomegalovirus. Herpes Virus. Human T-cell Lymphotropic Virus. Mycoplasma, and others) and endogenous proteins (collagen, proteoglycans, altered immunoglobulins) have been implicated as the causative agent which triggers an inappropriate host immune response. The end result is the production of an excessive inappropriate immune response directed against the host tissues (e.g., antibodies directed against Type II collagen, antibodies directed against the Fc position of autoloeous IgG (called "Rheumatoid Factor")). This further amplifies the immune response cartilage destruction are responsible for the progression of rheumatoid arthritis. In rheumatoid arthritis, the main presenting symptoms are pain, stiffness, swelling, and loss of function (Bennett J C. The etiology of rheumatoid arthritis. In Textbook of Rheumatology (Kelley W N, Harris E D, Ruddy S. Sledge C B. eds.) W B Saunders, Philadelphia pp 879-886. 1985).

The cytokines IL-1α, IL-1β, IL-4, 1L-8, IL-10, TNF-α, PDGF, FGF, OM-CSF, IFN-γ, TGF-β. IL-2 and IL-6 enhances the activity of fibroblast-like cells in the synovium, chondrocytes and macrophages, thereby releasing increased amounts of proteoglycans, neutral proteinases such as collagenases, transin and stromelysin. These factors cause the recruitment of osteoclast precursors, ultimately culminating in the destruction of bone and cartilage by the invading proliferative synovium. The destructive cascade is characterized physically by increased thinning of the cartilage layer, decreased proteoglycan synthesis, and diminished load-bearing capacity.

Several combination therapies have been described for treating RA. The combination of etanerecpt (TNFR:Fc fusion protein) and methotrexate (MTX) was used to treat persistently active RA and found to provide greater clinical benefit than methotrexate alone (Weinblatt et al., Jan. 28. 1999. NEJM 340 (4): 253-259). In another clinical trial, the anti-TNF-α chimeric mouse-human antibody, cA2 (infliximab; Remicade,) was given in combination with low-dose methotrexate to RA patients (Mani et al, 1998. Arthritis & Rheumatism 41(9): 1552-1563). Anti-CD4 mAb was found to prevent collagen-induced arthritis if administered before the onset of clinical disease in the CIA mouse model but was ineffective in treating established disease. Co-administration of anti-CD4 antibody with anti-TNF α/β antibody caused significantly greater reduction in paw swelling and joint erosion than that observed by optimal anti-TNF alone (Williams et al. 1994, PNAS 91: 2762-2766). For other references on combination therapies see Kremer (1998). Arthritis & Rheumatism 41: 1548-1551 and Williams (1998). Springer Semin. Immunopathol. 20:165-180.

Administration of many therapeutic agents rapidly induces adverse side effects, or events, including but not limited to fever, headache, nausea, vomiting, breathing difficulties and changes in blood pressure. These adverse events limit the amount of a drug or therapeutic compound that can be given, which in turn limits the therapeutic effectiveness that could be achieved with higher doses of the drug. Adverse events have also been associated with the initial administration of monoclonal antibodies directed to other cell surface molecules. A humanized anti-CD4 monoclonal antibody induced fever, chills, hypotension and chest tightness when given intravenously to psoriasis and rheumatoid arthritis patients (Isaacs, et al., 1997 Clin Exp Immunol. 110. 158-166). This treatment down-modulated expression of CD4 and caused a reduction in the number of circulating CD4-positive T cells.

### Summary of the Invention

The invention provides the use of an LFA-1 antagonist and a TNF-α antagonist in the preparation of a medicament for treating rheumatoid arthritis, wherein the LFA-1 antagonist is an anti-CD11a antibody, and the TNF-α antagonist is an immunoadhesin comprising at least a TNF-α binding portion of the p75 TNF-α receptor. Reference throughout this specification to a LFA-1 antagonist and/or a TNF-α antagonist should be construed accordingly.

The invention further provides a composition comprising an LFA-1 antagonist and a TNF-α antagonist, wherein the LFA-1 antagonist is an anti-CD11a antibody, and the TNF-α antagonist is an immunoadhesin comprising at least a TNF-α binding portion of the p75 TNF-α receptor.

The composition may further comprise a carrier, excipient or stabilizer. The composition may be formulated for administration via injection or infusion by intravenous, intraarterial, intraperitoneal, intramuscular, intralesional, intraarticular or topical administration to a mamma and the amount administered is a therapeutically effective amount. Alternatively, the composition may be formulated for injection directly into the afflicted cartilagenous region or joint.

The composition may be are extended-release composition containing the LFA-1 antagonist and the TNF-α antagonist. More specifically, the extended-release composition may be formulated in a microcncapsulation, a semi-permeable membrane of solid hydrophobic polymers, a biodegradable polymer(s), or a dispersion (*e.g.,* suspension or emulsion). More specifically, the semi-permeable membrane of solid hydrophoblic polymer is poly-lactic-co-glycolic acid (PLGA), and the biogradable polymer is cross-linked hyaluronic acid (HA). Alternatively, the extended-release composition further comprises a water-soluble polyvalent metal salt. More specifically, the polyvalent metal salt includes the salt formed from an alkaline earth metal and an inorganic or oreanic acid.

The LFA-1 antagonist and TNP-α antagonist may be administered in combination with an effective amount of cartilage growth factor. Optionally, the cartilage is present inside a mammal and the amount administered is a therapeutically effective amount. More specifically, the cartilage growth factor may be insulin-like growth factors (*e*.*g*., IGF-1, IGF-2), platelet-derived growth factor (PDGF), bone morphogenic proteins (BMPs), discuptors or down regulators of *c-myc* or *Bcl-2* expression, antisense RNA or DNA or disruption of associated promoter regions. Optionally the cartilage growth factor may be an agent which enhances the reparative response of intrinsic cartilage, such as through increasing the actual or potential proliferation of chondrocytes (*e*.*g*., basic fibroblast growth factor (bFGF)), or through the forced progression of cell differentiation cell cycle progression factors such as IGF's. TGF-β and epidermal growth factors (EGF). Optionally: the cartilage growth factor may be an agent which antagonizes the catabolism of cartilage (*e*.*g*., IL-1 receptor antagonist (IL-Ira), NO inhibitors).

The LFA-1 antagonist and TNF-α antagonist may be used for preventing the development or delaying the onset of rheumatoid arthritis in subjects genetically disposed or susceptible to developing rheumatoid arthritis. In a specific embodiment, the RA is juvenile RA and the subject is a juvenile (under age 16).

In a specific embodiment, the LFA-1 antagonist is anti-CD11a antibody and the TNF-α antagonist is etanercept. The therapeutic/optimal doses for anti-CD11a antibody hul124 and for etanercept are available from the drug product literature. The LFA-1 antagonist, and TNF-α antagonist may be formulated for administration with methotrexate. In a preferred embodiment of all aspects of the invention, it is preferred that the anti-CD11a antibody be a non-lymphocyte depleting, in particular, non-T cell depleting antibody. The anti-CD11a antibody, hul124, is non-T cell depleting. In more specific embodiments, anti-CD11a antibody is a human or humanized antibody or antibody fragment thereof, most preferably, the humanized antibody hu 1124 disclosed and claimed in U.S. patent 6.037,454. In a preferred embodiment of all aspects of the invention, the TNF-α antagonist is a TNF-α receptor - IgG Fc fusion protein such as etanercept.

In another embodiment, the present invention concerns a therapeutic kit, comprising the LFA-1 antagonist and TNF-α antagonist and a carrier, excipient and/or stabilizer (*e*.*g*. a buffer) in suitable packaging. The kit preferably contains instructions for using an LFA-1 antagonist and a TNF-α antagonist to treat rheumatoid arthritis.

### Brief Description of the Drawing

Figure 1 shows the effect of treatment with a combination of an LFA-1 antagonist and a TNF antagonist in reducing the incidence of clinical arthritis in animals (see Example 1).
Figure 2 shows the effect of treatment with either anti-murine CD11a antibody (M17) alone, or TNF antagonist (Enbrel) alone, on arthritis in DBA-1LacJ mice, as indicated by the mean clinical scores (see Example 2). Saline treatment served as a control.
Figure 3 shows the effect of treatment with anti-murine CD11a antibody (M 17) alone, or saline (control), on arthritis in DBA-1J mice, as indicated by the mean clinical scores (see Example 2).
Figure 4 shows the effectiveness of treatment with a combination of an LFA-1 antagonist (antibody M17) and a TNF antagonist (Enbrel), as compared to the individual antagonist alone, in reducing clinical arthritis in DBA-1LacJ mice (see Example 3).
Figure 5 shows the effectiveness of treatment with a combination of an LFA-1 antaeonist (antibody M17) and a TNF antagonist (Enbrel), as compared to the individual antagonist alone, in reducing clinical arthritis in DBA-1J mice (see Example 3).

### Detailed Description of the Preferred Embodiments

### 1. Definitions

Administration "in combination with" one or more further therapeutic agents includes simultaneous (concurrent) and consecutive administration in any order.

The term "antagonist" with respect to LFA-1 or TNF-α means a compound which is capable of, directly or indirectly, counteracting, reducing or inhibiting the biological activity of LFA-1 or TNF-α or activation of receptors therefor.

"ENBREL" (etanercept) is a dimeric fusion protein consisting of the extracellular ligand-binding portion of the human 75 kilodalton (p75) tumor necrosis factor receptor (TNFR) linked to the Fc portion of human IgG1. The Fc component of etanercept contains the CH2 domain, the CH3 domain and hinge region, but not the CH I domain of IgG1. Etanercept binds specifically to rumor necrosis factor (TNF) and blocks its interaction with cell surface TNF receptors. It inhibits the activity of TNF.

"Biological" activity refers to a biological function (either inhibitory or stimulatory) caused by a native or naturally-occurring molecule, such as LFA-1 or TNF-α, other than the ability to serve as an antigen in the production of an antibody against an antigenic epitope possessed by a native or naturally-occurring polypeptide of the invention. Similarly, an "immunological' activity refers to the ability to serve as an antigen in the production of an antibody against an antigenic epitope possessed by the antigen. Some of the biological activities of TNF-α and LFA-1 are described in the background and throughout the specification.

"Carriers" as used herein include pharmaceutically acceptable carriers, excipients, or stabilizers which are nontoxic to the cell or mammal being exposed thereto at the dosages and concentrations employed. Often the physiologically acceptable carrier is an aqueous pH buffered solution. Examples of physiologically acceptable carriers include buffers such as phosphate, citrate, and other organic acids; antioxidants including ascorbic acid; low molecular weight (less than about 10 residues) polypeptide; proteins, such as serum albumin, gelatin, or immunoglobulins; hydrophilic polymers such as polyvinylpyrrolidone: amino acids such as glycine, glutamine, asparagine, arginine or lysine; monosaccharides, disaccharides, and other carbohydrates including glucose, mannose, or dextrins: chelating agents such as EDTA: sugar alcohols such as mannitol or sorbitol: salt-forming counterions such as sodium: and/or nonionic surfactants such as TWEEN™, polyethylene glycol (PEG), and PLURONICS™.

"Cartilage growth factor" as used herein, refers to agent(s) other than an LFA-1 antagonist or a TNF antagonists which cause, induce or result in an improvement in the condition of or protection from initial or continued destruction of cartilage subject to damage by either by injury or a degenerative cartilagenous disorder. Such cartilage growth factors include insulin-like growth factors (*e*.*g*., IGF-1, IGF-2), platelet-derived growth factor (PDGF), bone morphogenic proteins (BMPs), disruptors or down regulators of *c-myc* or *Bcl-2* expression, antisense RNA or DNA or disruption of associated promotor regions. Optionally the cartilage growth factor may be an agent which enhances the reparative response of intrinsic cartilage, such as through increasing the actual or potential proliferation of chondrocytes (*e*.*g*., basic fibroblast growth factor (bFGF)), or through the forced progression of cell differentiation cell cycle progression factors such as IGF's, TGF-β and epidermal growth factors (EGF).

A further subset of molecules which fall under the above definition of "cartilage growth factor" are agents which antagonize the catabolism of cartilage or a "cartilage catabolism antagonist." Cartilage catabolism antagonists may be defined as those agents which inhibit, attenuate or otherwise block the activity or effect of molecules that are associated with or aggravate cartilage destruction. For example, IL-1α and nitric oxide (NO) are agents known to be associated with cartilage destruction. Thus, inhibitors of IL-1α (*e*.*g*., IL-1ra) and NO production would be considered "cartilage catabolism antagonists. Moreover, antagonists of chondrocyte catabolism (*e*.*g*., sodium pentosan polysulfate) would also be considered cartilage catabolism antagonists.

"Chronic" administration refers to administration of the agent(s) in a continuous mode as opposed to an acute mode, so as to maintain the initial therapeutic effect (activity) for an extended period of time. "Intermittent" administration is treatment that is done not consecutively without interruption, but rather is cyclic in nature.

A "conditioning dose" is a dose which attenuates or reduces the frequency or the severity of first dose adverse side effects associated with administration of a therapeutic compound. The conditioning dose may be a therapeutic dose, a sub-therapeutic dose, a symptomatic dose or a sub-symptomatic dose. A therapeutic dose is a dose which exhibits a therapeutic effect on the patient and a sub-therapeutic dose is a dose which dose not exhibit a therapeutic effect on the patient treated. A symptomatic dose is a dose which induces at least one adverse effect on administration and a sub-symptomatic dose is a dose which does not induce an adverse effect.

Rheumatoid arthritis (RA) is a systemic, chronic, autoimmune disorder characterized by symmetrical synovitis of the joint and typically affects small and large diarthroid joints alike. As RA progresses, symptoms may include fever, weight loss, thinning of the skin, multiorgan involvement, scleritis, corneal ulcers, the formation of subcutaneous or subperiosteal nodules and even premature death. The symptoms of RA often appear during youth and can include vasculitis, atrophy of the skin and muscle, subcutaneous nodules, lymphadenopathy, splenomegaly, leukopaenia and chronic anaemia.

The term "effective amount" refers to the minimum concentrations of an LFA-1 antagonist and a TNF antagonist which cause, induce or result in either a detectable or measurable improvement or repair in damaged cartilage or a measurable protection from the continued or induced cartilage destruction in an isolated sample of cartilage matrix. For example, the inhibition of release of free proteoglycan from the cartilage tissue.

A "therapeutically effective amount" refers to the minimum concentrations (amount) of an LFA-1 antagonist and a TNF antagonist administered to a mammal that are effective in at least attenuating a pathological symptom (*e*.*g*. causing, inducing or resulting in a detectable / measurable improvement: lessen the severity, extent or duration of symptoms) which occurs as a result of RA.

The symptoms of RA are described below. For example, the therapeutically effective amount is effective in causing, inducing or resulting in either a detectable / measurable improvement or repair in damaged particular cartilage or causing, inducing or resulting in a measurable protection from the continued or initial cartilage destruction, improvement in range of motion, reduction in pain, *etc*.).

In treating rheumatoid arthritis (RA) in humans, the criteria for evaluating extent of or improvement in the disease may include for example, assessment of the number of tender and swollen joints, patient and physician global assessment (e.g., at 3 and 6 months from initiation of treatment), morning stiffness, pain, increased functional status (e.g., through a Health Assessment Questionnaire), disability, structural damage, and acute phase reactant. Preferably, the amounts of LFA-1 antagonist and TNF antagonist are effective to achieve in the patient, at least a 20% improvement in at least one of the preceding criteria, more preferably at least 30%, even more preferably, at least 40% or 50%, most preferably at least 75% improvement compared to the control or placebo treated patient. Alternatively, an improvement in at least one grade in clinical scores. e.g., in the Paulus criteria is considered effective treatment herein.

Disability and acute phase reactants are taught in Felson, DT et al. 1993. The American College of Rheumatology Preliminary Core Set of Disease Activity for Rheumatoid Arthritis Clinical Trials. Arthritis and Rheumatism. 36 (6): 729-740: and Felson, DT et al. 1995. The American College of Rheumatology Preliminary Definition of Improvement in Rheumatoid Arthritis. Disease, Arthritis and Rheumatism. 38 (40): 1.9. Structural damage can be evaluated by radiography which can reveal slowing X-ray progression of the disease based on a validated radiographic index such as the Larsen or modified Sharp index. One can also evaluate radiographically to determine if treatment prevents the formation of new joint erosions or slows the progression. Other methodologies could be employed such as magnetic resonance imaging, ultrasonagraphy, and NMR. The American College of Rheumatology (ACR) response criteria or alternatively, the Paulus criteria are well known criteria used in evaluating drug efficacy in treating rheumatoid arthritis. ACR criteria is based on tender joint count and swollen joint count; (1) patient pain assessment, (2) patient global assessment, (3) physician global assessment, (4) patient self-assessed disability, and (5) acute-phase reactant (ESR or CRP). The Paulus Criteria relies on improvement in at least four of the following: Tender joint score: Swollen joint score; Morning stiffness: Patient assessment of disease severity (5 point scale): Physician assessment of disease severity (5 point scale): and ESR.

The term "extended-release" or "sustained-release" formulations in the broadest possible sense means a formulation of active LFA-1 antagonist or TNF antagonist polypeptide resulting in the release or activation of the active polypeptide for a sustained or extended period of time or at least for a period of time which is longer than if the polypeptide was made available *in vivo* in the native or unformulated state. Optionally, the extended-release formulation occurs at a constant rate and/or results in sustained and/or continuous concentration of the active polypeptide. Suitable extended release formulations may comprise microencapsulation, semi-permeable matrices of solid hydrophobic polymers, biogradable polymers, biodegradable hydrogels, suspensions or emulsions (*e*.*g*., oil-in-water or water-in-oil). Optionally, the extended-release formulation comprises poly-lactic-co-glycolic acid (PLGA) and can be prepared as described in Lewis. "Controlled Release of Bioactive Agents form Lactide/Glycolide polymer," in Biodegradable Polymers as Drug Delivery Systems, M. Chasin & R. Langeer. Ed (Marcel Dekker. New York), pp. 1-41. Optionally, the extended-release formulation is stable and the activity of the LFA-1 antagonist or TNF antagonist does not appreciably diminish with storage over time. More specifically, such stability can be enhanced through the presence of a stabilizing agent such as a water-soluble polyvalent metal salt.

The term "immunoadhesin" refers to a chimeric molecule which is a fusion of a ligand binding moiety, such as a receptor extracellular domain, with an immunoglobulin or a particular region of an immunoglobulin. For a bivalent form of an immunoadhesin, such a fusion could be to the Fc region of an IgG molecule. In a particularly preferred embodiment, the immunoglobulin fusion includes the hinge, CH2 and CH3, or the hinge, CH1, CH2 and CH3 regions of an IgG1 molecule. For the production of immunoglobulin fusions see also US Patent No. 5,428,130. As used herein, the term "immunoadhesin" designates antibody-like molecules which combine the binding specificity of a heterologous protein (an "adhesin") with the effector functions of immunoglobulin constant domains. Structurally, the immunoadhesins comprise a fusion of an amino acid sequence with the desired binding specificity which is other than the antigen recognition and binding site of an antibody (*i*.*e*., is "heterologous"), and an immunoglobulin constant domain sequence. The adhesin part of an immunoadhesin molecule typically is a contiguous amino acid sequence comprising at least the binding site of a receptor or a ligand. The immunoglobulin constant domain sequence in the immunoadhesin may be obtained from any immunoglobulin, such as IgG-1, IgG-2, IaG-3, or IgG-4 subtypes, IgA (including IsA-1 and IgA-2). IgE, IgD or IgM.

A "liposome" is a small vesicle composed of various types of lipids, phospholipids andior surfactant which is useful for delivery of a drug to a mammal. The components of the liposome are commonly arranged in a bilayer formation, similar to the lipid arrangement of biological membranes.

"Mammal" for purposes of treatment refers to any animal classified as a mammal, including humans, domestic and farm animals, and zoo, sports, or pet animals, such as dogs, horses, cats, cattle, *etc*. Preferably, the mammal is human.

The "pathology" of a degenerative cartilagenous disorder includes all physiological phenomena that compromise the well-being of the patient. This includes, without limitation, cartilage destruction, diminished cartilage repair, abnormal or uncontrollable cell growth, antibody production, auto-antibody production, complement production and activation, interference with the normal functioning of neighboring cells, release of cytokines or other secretory products at abnormal levels, suppression or aggravation of any inflammatory or immunological response, infiltration of inflammatory cells (neutrophilic, eosinophilic, monocytic, lymphocytic) into tissue spaces, *etc*.

A "small molecule" is defined herein to have a molecular weight below about 600 daltons, and is generally an organic compound.

By "solid phase" is meant a non-aqueous matrix to which the compound of the present invention can adhere. Examples of solid phases encompassed herein include those formed partially or entirely of glass (*e*.*g*., controlled pore glass), polysaccharides (*e*.*g*., agarose), polyacrylamides, polystyrene, polyvinyl alcohol and silicones. In certain embodiments, depending on the context, the solid phase can comprise the well of an assay plate; in others it is a purification column (*e*.*g*., an affinity chromatography column). This term also includes a discontinuous solid phase of discrete particles, such as those described in U.S. Patent No. 4,275.149.

"Treatment" is an intervention performed with the intention of preventing the development or altering the pathology of a disorder. Accordingly, "treatment" refers to both therapeutic treatment and prophylactic or preventative measures, wherein the object is to prevent, slow down or lessen the severity, extent or duration of symptoms, or delay the onset of (*e*.*g*., in subjects predisposed to develop RA due to genetic make-up or other risk factors) the targeted pathological condition or disorder. "Treating" a disease, disorder, condition or cell population includes therapy and prophylactic treatment on an acute short term basis and on a chronic long-term basis. Those in need of treatment include those already with the disorder as well as those in which the disorder is to be prevented. Treatment is successful if it results in a detectable or measurable improvement in at least one symptom of the disorder being treated (consistent with the definition of "therapeutically effective amount" above).

A therapeutic agent may directly decrease or increase the magnitude of response of a pathological component of the disorder, or render the disease more susceptible to treatment by other therapeutic agents, *e*.*g*. antibiotics, antifungals, anti-inflammatory agents, chemotherapeutics, *etc*.

### 11. Modes for Carrying Out the Invention

### A. Antagonist

Anti-CD11a antibodies include, e.g.. MHM24 [Hildreth et al., Eur, J. Immunol., 13: 202-208 (1983)], R3.1 (IgG1) [R. Rothlein, Boehringer Ingelheim Pharmaceuticals. Inc., Ridgefield, CT], 25-3 (or 25.3), an IgG1 available from Immunotech. France [Olive et al., in Feldmann. ed., Human T cell Clones. A new Approach to Immune Regulation. Clifton. NJ. Humana. 1986 p. 173]. KBA (IgG2a) (Nishimura et al., Cell. Immunol., 107: 32 (1987): Nishimura et al., ibid., 94: 122 (1985)). M7/15 (IgG2b) [Springer et al., Immunol. Rev., 68: 171 (1982)]. IOT16 [Vermot Desroches et al., Scand. J. Immunol., 33: 277-286 (1991)]. SPVL7 [Vermot Desroches *er al*., *supra*], and M17 (IgG2a), available from ATCC. which are rat anti-murine CD11a antibodies. Preferred anti-CD11a antibodies are the humanized antibodies described in U.S. Patent 6,037,454. It is also generally preferred that the anti-CD11a antibodies are not T-cell depleting antibodies, that is, that the administration of the anti-CD11a antibody does not reduce the level of circulating T-cells.

Molecular cloning has demonstrated the existence of two distinct types of TNF receptors (TNFR) with apparent molecular masses of 55kD (type I) (Schall et al., (1990) Cell 61:361) and 75 kD (type 2) (Smith ct al., (1990) Science 248:1019), each of which naturally binds to both TNF-α and TNF-β (Loetscher et al., (1990) Cell, 61:351; Shall et al. (1990) Cell. 61:361: Kohno et al., 1990) Proc. Natl. Acad. Sci. USA 87:8331). The extracellular portions of both receptors are found naturally as soluble TNF binding proteins (Kohno et al., supra). TNF antagonists have been created which block the deleterious effect of TNF in various immune and inflammatory events (Peppel et al., (1991) J. Exp. Med.. 174:1483-1489: Ulich (1993) Am. J. Path., 142:1335-1338: Howard. O.M.Z.. (1993) Proc. Natl. Acad. Sci. USA 90:2335-2339: Wooley. P.H.. (1993) J. Immunol. 151:6602-6607). One such antagonist (Werner et al., (1991) J. Cell. Biochem. Abstracts. 20th annual meeting. p. 115) combines the extracellular domain of human 55 kD type 1 TNFR with a portion of the hinge and Fc regions of human immunoglobulin G 1 heavy chain. Another such antagonist (Mohler et al.. (1993) J. Immunol. 151:1548-1561) combines the extracellular domain of human 75 kD type 2 TNFR with a portion of the hinge and Fc regions of human immunoglobulin G1 heavy chain. U.S. patents 5,482.130 and 5.514.582 describe these molecules.

The TNF-α antagonist, is an immunoadhesin containing at least a TNF-α binding portion of a 75 TNF-α receptor. Preferred immunoadhesins are disclosed in US 5.605.690 and 5.712.155. for example.

In preferred embodiments, the TNF-α antagonist is a TNF-α receptor - IgG Fc fusion protein, such as ENBREL (Immunex) and the LFA-1 antagonist is an anti-CD11a antibody, preferably a non T-cell depicting anti-CD11a antibody such as hul 124 (XOMA/Genentech).

The LFA-1 antagonist and the TNF-α antagonist may be administered in amounts conventionally used for these compounds. The compounds may be adminstered at a molar ratio of about 1:1000 to about 1000:1, or about 100:1 to about 1:100. or about 1:10 to about 10:1, or in a ratio of about 1:5 to about 5:1, or even at a ratio of about 1:1.

### B. Administration

The combination of compounds of the present invention are administered to a mammal, preferably a human, in accord with known methods, such as intravenous administration as a bolus or by continuous infusion over a period of time, by intramuscular, intraperitoneal, intracerebrospinal, subcutaneous, intra-articular, intrasynovial, intrathecal, oral, topical, intralesional, intraarticular or inhalation (intranasal, intrapulmonary, aerosolized) routes and by sustained release or extended-release means. Optionally the active compound or formulation is injected directly into an afflicted cartilagenous region or articular joint.

Administration of the LFA-1 antagonist and TNF antagonist, separately or together, may be in dosage amounts for each compound varying from about 10 ng/kg to up to 100 mg/kg of mammal body weight or more per day, preferably about I µg/kg/day to 10 mg/kg/day, depending upon the route of administration. Guidance as to particular dosages and methods of delivery is provided in the literature for each of the compounds. For example. ENBREL is currently recommended at a dosage of 25 mg for adult humans, twice weekly as subcutaneous injection given at least 72-96 hours apart. In one case, up to 62 mg ENBREL has been administered to an adult subcutaneously (SC) twice weekly for 3 weeks without producing adverse effects (see PDR). The recommended dose of ENBREL for pediatric patients ages 4 to 17 years with active polyarticular-course JRA is 0.4 mg/kg (up to a maximum of 25 mg per dose) given twice weekly as a subcutaneous injection 72-96 hours apart. Methotrexate (see Weinblatt et al., Jan. 28, 1999; Mani et al. 1998, supra), glucocorticoids, salicylates, nonsteroidal anti-inflammatory drugs (NSAIDs), or analgesics may be continued during treatment with ENBREL. An LFA-1 antagonist, humanized anti-CD11a antibody hul124, can be administered at a dosage range of between 0.3 mg/kg to 6 mg/kg. LFA-1 antagonism may allow the use of lower doses of drugs for TNF antagonism, and vice versa, to attain the same or better efficacy but with reduced clinical adverse events including but not limited to fever, chills, infection, sepsis and anemia. Thus, in the treatment methods of the present invention, dosages of one or both of the antagonists can be reduced to minimize any toxicity or adverse events that can occur with administration of the normal or recommended dose for either antagonist alone. For example, when used together in the present treatment methods, the aforementioned dosages for ENBREL and hul124 can be reduced, especially in the treatment of juveniles (e.g., for Juvenile RA).

The appropriate dosages of the compounds of the invention will depend on the type of disease to be treated, as defined above, the severity and course of the disease, whether the agent is administered for preventive or therapeutic purposes, previous therapy, the patient's clinical history and response to the compound and the discretion of the attending physician. The determination of the appropriate dosage or route of administration is well within the skill of an ordinary physician. Animal experiments provide reliable guidance for the determination of effective doses for human therapy. Interspecies scaling of effective doses can be performed following the principles laid down by Mordenti. J. and Chappell. W. "The use of interspecies scaling in toxicokinetics" in Toxicokinetics and New Drug Development. Yacobi et al., Eds.. Pergamon Press. New York 1989, pp. 42-96.

The doses may be administered according to any time schedule which is appropriate for treatment of the disease or condition. For example, the dosages may be administered on a daily, weekly, biweekly or monthly basis in order to achieve the desired therapeutic effect and reduction in adverse effects. The compound is suitably administered to the patient at one time or over a series of treatments. The dosages can be administered before, during or after the development of the disorder.

The specific time schedule can be readily determined by a physician having ordinary skill in administering the therapeutic compound by routine adjustments of the dosing schedule within the method of the present invention.

The dosing schedule may include a first conditioning dose of one or both antagonists followed by a second higher or therapeutic dose of the antagonists, to condition the mammal to tolerate increasing or higher doses of the therapeutic compounds. This dosing schedule allows one to reduce the occurrence of adverse effects which arise from the initial administration and subsequent administrations of the therapeutic compound (see WO 0056363). Although some adverse effects such as fever, headache, nausea, vomiting, breathing difficulties, myalgia, chills and changes in blood pressure may still be observed, the frequency and/or severity of these adverse effects may be reduced relative to administration using conventional dosing schedules such as daily administration of equal doses of a therapeutic compound.

For example, depending on the type and severity of the disease, about 1 µg/kg to 15 mg/kg (*e.g*. 0.1-20mg/kg) of each of the compounds of the invention is an initial candidate dosage for administration to the patient, whether, for example, by one or more separate administrations, or by continuous infusion. A typical daily dosage might range from about 1 µg/kg to 100 mg/kg or more, depending on the factors mentioned above. A preferred dose of about 0.1-30 mg/kg is particularly useful for antagonists that are antibodies or fragments thereof. For repeated administrations over several days or longer, depending on the condition, the treatment is sustained until a desired suppression of disease symptoms occurs. However, other dosage regimens may be useful. The progress of this therapy is easily monitored by conventional techniques and assays. The compounds may be administered concurrently or sequentially or a combination thereof. For example, the TNF-α antagonist may be dosed initially and then followed by administration of the LFA-1 antagonist. Alternatively, the LFA-1 antagonist may be dosed initially and then followed by administration of the TNF-α antagonist. The antagonists may be dosed, for example, daily or every other day for a period of a few (2-4) days or for several (2-6) weeks during a single course of therapy. As noted above, repeated courses of therapy may be administered until the desired suppression of disease or disorder are suppressed.

ENBREL is supplied as a sterile, white, preservative-free, lyophilized powder for parenteral administration after reconstitution with 1 ml of the supplied Sterile Bacteriostatic Water for Injection. USP (containing 0.9% benzyl alcohol).

It is believed that the administration of an LFA-1 antagonist and a TNF-α antagonist provides an improved treatment relative to treatment with either one of the individual compounds alone. That is, treatment with both compounds provides a reduction in the incidence of disease or disorder symptoms relative to a control, for example, which is lower than the reduction in disease or disorder incidence relative to a control, for administration of either compound alone. Such improved efficacy is evidence of a synergistic action of the compounds of the invention.

The synergism is particularly surprising for LFA-1 antagonists. i.e. anti-CD11a antibodies, which do not deplete T-cells.

The LFA-1 antagonist and TNF-α antagonist can be administered concurrently with other therapy. For example, a patient being treated for RA can be administered both these antagonists in conjunction with or in addition to conventional drugs used in RA such methotrexate, glucocorticoids, salicylates, nonsteroidal anti-inflammatory drugs (NSAIDS), or analgesics.

### C. Compositions

The compounds of the invention can be administered in the form of pharmaceutical compositions. Additionally, lipofections or liposomes can also be used to deliver the LFA-1 antagonist or TNF antagonist into cells and the target area.

Therapeutic formulations of the active molecules employable with the invention are prepared for storage by mixing the active molecule having the desired degree of purity with optional pharmaceutically acceptable carriers, excipients or stabilizers (Remington's Pharmaceutical Sciences 16th edition. Osol. A. Ed. [1980]). Such therapeutic formulations can be in the form of lyophilized formulations or aqueous solutions. Acceptable carriers, excipients, or stabilizers are nontoxic to recipients at the dosages and concentrations employed, and include buffers such as phosphate, citrate, and other organic acids: antioxidants including ascorbic acid and methionine: preservatives (such as octadecyldimethylbenzyl ammonium chloride; hexamethonium chloride: benzalkonium chloride, benzethonium chloride; phenol, butyl or benzyl alcohol; alkyl parabens such as methyl or propyl paraben: catechol; resorcinol; cyclohexanol: 3-pentanol: and *m*-cresol); low molecular weight (less than about 10 residues) polypeptides; proteins, such as serum albumin, gelatin, or immunoglobulins: hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol: salt-forming counter-ions such as sodium; metal complexes (*e.g.* Zn-protein complexes); and/or non-ionic surfactants such as TWEEN^{®}. PLURONICS^{®} or polyethylene glycol (PEG).

In order for the formulations to be used *in vivo* administration, they must be sterile. The formulation may be readily rendered sterile by filtration through sterile filtration membranes, prior to or following lyophilization and reconstitution. The therapeutic compositions herein generally are placed into a container having a sterile access port, for example, an intravenous solution bag or vial having a stopper pierceable by a hypodermic injection needle.

The formulations used herein may also contain more than one active compond as necessary for the particular indication being treated, preferably those with complementary activities that do not adversely affect each other. Alternatively, or in addition, the composition may comprise a cytotoxic agent, cytokine or growth inhibitory agent. Such molecules are present in combinations and amounts that are effective for the intended purpose.

The LFA-1 antagonist or TNF-α antagonist molecules by also be prepared by entrapping in microcapsules prepared, for example by coacervation techniques or by interfacial polymerization, for example, hydroxymethylcellulose or gelatin-microcapsules and poly-(methylmethacrylate) microcapsules, respectively. Such preparations can be administered in colloidal drug delivery systems (for example, liposomes, albumin microspheres, microemulsions, nano-particles and nanocapsules) or in macroemulsions. Such techniques are disclosed in Remington's Pharmaceutical Sciences, 16th Edition (or newer). Osol A. Ed. (1980).

Where sustained-release or extended-release administration of the an LFA-1 antagonist or a TNF-A antagonist is desired in a formulation with release characteristics suitable for the treatment of any disease or disorder requiring administration of such polypeptides, microencapsulation is contemplated. Microencapsulation of recombinant proteins for sustained release has been successfully performed with human growth hormone (rhGH), interferon-α, -β, -γ (rhIFN-α,-β,-γ), interleukin-2, and MN rgp120. Johnson et al., Not. Med. 2: 795-799 (1996); Yasuda. Biomed Ther. 27: 1221-1223 (1993); Hora et al.. Bio/Technology 8: 755-758 (1990): Cleland. "Design and Production of Single Immunization Vaccines Using Polylactide Polyglycolide Microsphere Systems" in Vaccine Design: The Subunit and Adjuvant Approach, Powell and Newman, eds., (Plenum Press: New York, 1995), pp. 439-462; WO 97/03692, WO 96/40072. WO 96/07399 and U.S. Pat. No. 5,654,010.

Suitable examples of sustained-release preparations include semipermeable matrices of solid hydrophobic polymers containing the active molecule, which matrices are in the form of shaped articles. *e.g*. films, or microcapsules. Examples of sustained-release matrices include one or more polyanhydrides (e.g.. U.S.P. 4,891,225; 4,767,628), polyesters such as polyglycolides, polylactides and polylactide-co-glycolides (*e*.*g*., U.S.P. 3,773.919: U.S.P. 4.767,628: U.S.P. 4.530.840: Kulkarni et al., Arch. Surg. 93: 839 (1966)), polyamino acids such as polylysine, polymers and copolymers of polyethylene oxide, polyethylene oxide acrylates, polyacrylates, ethylene-vinyl acetates, polyamides, polyurethanes, polyorthoesters, polyacetylnitriles, polyphosphazenes, and polyester hydrogels (for example, poly(2-hydroxyethyl-methacrylate), or poly(vinylalcohol)), cellulose, acyl substituted cellulose acetates, non-degradable polyurethanes, polystyrenes, polyvinyl chloride, polyvinyl fluoride, poly(vinylimidazole), chlorosulphonated polyolefins, polyethylene oxide, copolymers of L-glutamic acid and γ-ethyl-L-glutamate, non-degradable ethylene-vinyl acetate, degradable lactic acid-glycolic acid copolymers such as the LUPRON DEPOT^{™} (injectable microspheres composed of lactic acid-glycolic acid copolymer and leuprolide acetate), and poly-D-(-)-3-hydroxybutyric acid. While polymers such as ethylene-vinyl acetate and lactic acid-glycolic acid enable release of molecules for over 100 days, certain hydrogels release proteins for shorter time periods. Additional non-biodegradable polymers which may be employed are polyethylene, polyvinyl pyrrolidone, ethylene vinylacetate, polyethylene glycol, cellulose acetate butyrate and cellulose acetate propionate.

Alternatively, sustained release formulations may be composed of degradable biological materials. Biodegradable polymers are attractive drug formulations because of their biocompatibility, high responsibility for specific degradation, and ease of incorporating the active drug into the biological matrix. For example, hyaluronic acid (HA) may be crosslinked and used as a swellable polymeric delivery vehicle for biological materials. U.S.P. 4,957,744; Valle et al., Polym. Mater. Sci. Eng. 62: 731-735 (1991). HA polymer grafted with polyethylene glycol has also been prepared as an improved delivery matrix which reduced both undesired drug leakage and the denaturing associated with long term storage at physiological conditions. Kazuteru. M., J. Controlled Release 59:77-86 (1999). Additional biodegradable polymers which may be used are poly(caprolactone), polyanhydrides, polyamino acids, polyorthoesters, polycyanoacrylates, poly(phosphazines), poly(phosphodiesters), polyesteramides, polydioxanones, polyacetals, polyketals, polycarbonates, polyorthocarbonates, degradable and nontoxic polyurethanes, polyhydroxylbutyrates, polyhydroxyvalerates, polyalkylene oxalates, polyalkylene succinates, poly(malic acid), chitin and chitosan.

Alternatively, biodegradable hydrogels may be used as controlled release delivery vehicles for biological materials and drugs. Through the appropriate choice of macromers, membranes can be produced with a range of permeability, pore sizes and degradation rates suitable for a wide variety of biomolecules.

Alternatively, sustained-release delivery systems for biological materials and drugs can be composed of dispersions. Dispersions may further be classified as either suspensions or emulsions. In the context of delivery vehicles for biological materials, suspensions are a mixture of very small solid particles which are dispersed (more or less uniformly) in a liquid medium. The solid particles of a suspension can range in size from a few nanometers to hundreds of microns, and include microspheres, microcapsules and nanospheres. Emulsions, on the other hand, are a mixture of two or more immiscible liquids held in suspension by small quantities of emulsifiers. Emulsifiers form an interfacial film between the immiscible liquids and are also known as surfactants or detergents. Emulsion formulations can be both oil in water (o/w) wherein water is in a continuous phase while the oil or fat is dispersed, as well as water in oil (w/o), wherein the oil is in a continuous phase while the water is dispersed. One example of a suitable sustained-release formulation is disclosed in WO 97/25563. Additionaly, emulsions for use with biological materials include multiple emulsions, microemulsions, microdroplets and liposomes. Microdroplets are unilamellar phospholipid vesicles that consist of a spherical lipid layer with an oil phase inside. *E.g.*, U.S.P. 4,622,219 and U.S.P. 4.725.442. Liposomes are phospholipid vesicles prepared by mixing water-insoluble polar lipids with an aqueous solution.

Alternatively, the sustained-release formulations of LFA-1 antagonist or TNF-α antagonist may be developed using poly-lactic-coglycolic acid (PLGA), a polymer exhibiting a strong degree of biocompatibility and a wide range of biodegradable properties. The degradation products of PLGA. lactic and glycolic acids, are cleared quickly from the human body. Moreover, the degradability of this polymer can be adjusted from months to years depending on its molecular weight and composition. For further information see Lewis, "Controlled Release of Bioactive Agents from Lactide/Glycolide polymer." in Biogradable Polymers as Drug Delivery Systems M. Chasin and R. Langeer, editors (Marcel Dekker: New York. 1990), pp. 1-41.

When encapsulated polypeptides remain in the body for a long time, they may denature or aggregate as a result of exposure to moisture at 37°C, resulting in a loss of biological activity and possible changes in immunogenicity. Rational strategies can be devised for stabilization depending on the mechanism involved. For example, if the aggregation mechanism is discovered to be intermolecular S-S bond formation through thio-disulfide interchange, stabilization may be achieved by modifying sulfhydryl residues, lyophilizing from acidic solutions, controlling moisture content, using appropriate additives, and developing specific polymer matrix compositions.

The encapsulated polypeptides or polypeptides in extended-release formulation may be imparted by formulating the polypeptide with a "water-soluble polyvalent metal salts" which are non-toxic at the release concentration and temperature. Exemplary "polyvalent metals" include alkaline earth metals (*e*.*g*., Ca²⁺, Mg²⁺, Zn²⁺, Fe²⁺, Fe³⁺, Cu²⁺, Sn²⁺, Sn⁴⁺, Al²⁺ and Al³⁺). Exemplary anions which form water soluble salts with the above polyvalent metal cations include those formed by inorganic acids and/or organic acids. Such water-soluable salts have a solubility in water (at 20°C) of at least about 20 mg/ml, alternatively 100 mg/ml, alternatively 200 mg/ml.

Suitable inorganic acids that can be used to form the "water soluble polyvalent metal salts" include hydrochloric, sulfuric, nitric, thiocyanic and phosphoric acid. Suitable organic acids that can be used include aliphatic carboxylic acid and aromatic acids. Aliphatic acids within this definition may be defined as saturated or unsaturated C₂₋₉ carboxylic acids (*e*.*g*., aliphatic mono-, di- and tri-carboxylic acids). For example, exemplary monocarboxylic acids within this definition include the saturated C₂₋₉ monocarboxylic acids acetic, proprionic, butyric, valeric, caproic, enanthic, caprylic pelargonic and capryonic, and the unsaturated C₂₋₉ moncarboxylic acids acrylic, propriolic methacrylic, crotonic and isocrotonic acids. Exemplary dicarboxylic acids include the saturated C₂₋₉ dicarboxylic acids malonic, succinic, glutaric, adipic and pimelic, while unsaturated C₂₋₉ dicarboxylic acids include maleic, fumaric, citraconic and mesaconic acids. Exemplary tricarboxylic acids include the saturated C₂₋₉ tricarboxylic acids tricarballylic and 1,2,3-butanetricarboxylic acid. Additionally, the carboxylic acids of this definition may also contain one or two hydroxyl groups to form hydroxy carboxylic acids. Exemplary hydroxy carboxylic acids include glycolic, lactic, glyceric, tartronic, malic, tartaric and citric acid. Aromatic acids within this definition include benzoic and salicylic acid.

Commonly employed water soluble polyvalent metal salts which may be used to help stabilize the encapsulated polypeptides of this invention include, for example: (1) the inorganic acid metal salts of halides (*e*.*g*., zinc chloride, calcium chloride), sulfates, nitrates, phosphates and thiocyanates: (2) the aliphatic carboxylic acid metal salts calcium acetate, zinc acetate, calcium proprionate, zinc glycolate, calcium lactate, zinc lactate and zinc tartrate: and (3) the aromatic carboxylic acid metal salts of benzoates (*e*.*g*., zinc benzoate) and salicylates.

### D. Therapeutic Utility

The invention relates to the treatment of rheumatoid arthritis. RA refractory to or intolerant of methotrexate can also be treated with LFA-1 and TNF-α antagonists as described herein.

Rheumatoid arthritis (RA) is a chronic, systemic autoimmune inflammatory disease that mainly involves the synovial membrane of multiple joints with resultant injury to the articular cartilage. The pathogenesis is T lymphocyte dependent and is associated with the production of rheumatoid factors, auto-antibodies directed against self IgG. with the resultant formation of immune complexes that attain high levels in joint fluid and blood. These complexes in the joint may induce the marked infiltrate of lymphocytes and monocytes into the synovium and subsequent marked synovial changes: the joint space/fluid is infiltrated by similar cells with the addition of numerous neutrophils. Tissues affected are primarily the joints, often in symmetrical pattern. However, extra-articular disease also occurs in two major forms. One form is the development of extra-articular lesions with ongoing progressive joint disease and typical lesions of pulmonary fibrosis, vasculitis, and cutaneous ulcers. The second form of extra-articular disease is the so called Felty's syndrome which occurs late in the RA disease course, sometimes after joint disease has become quiescent, and involves the presence of neutropenia, thrombocytopenia and splenomegaly. This can be accompanied by vasculitis in multiple organs with formations of infarcts, skin ulcers and gangrene. Patients often also develop rheumatoid nodules in the subcutis tissue overlying affected joints: the nodules late stage have necrotic centers surrounded by a mixed inflammatory cell infiltrate. Other manifestations which can occur in RA include: pericarditis, pleuritis, coronary arteritis, intestitial pneumonitis with pulmonary fibrosis, keratoconjunctivitis sicca, and rheumatoid nodules.

### E. Assays/models

Assays and animal models are useful to evaluate the activity of the combination of compounds used in the methods of the invention. Some assays and models useful in assessing the effectiveness of the compounds in the treatment of RA are described below.

### Collagen induced arthritis assay/model

Rheumatoid arthritis (RA) is an immune disorder which appears to involve production of auto-antibodies. Antibodies to a protein expressed exclusively in cartilage, namely type II collagen, are present in the synovial fluid of some RA patients. Trentham, D.E et al., Arthrit. Rheum. 24: 1363-9 (1981). However, these antibodies are not necessarily the cause of the disease, but rather may be secondary to the inflammation. Injection of type II collagen into animals creates a specific immune reaction within synovial joints.

The collagen-induced arthritis (CIA) model is considered a suitable model for studying potential drugs or biologics active in human arthritis because of the many immunological and pathological similarities to human RA, the involvement of localized major histocompatibility, complete class-II-restricted T helper lymphocyte activation, and the similarity of histological lesions. Features of this CIA model which are similar to that found in RA patients include: erosion of cartilage and bone at joint margins (as can be seen in radiographs) proliferative synovitis, symmetrical involvement of small and medium-sized peripheral joints in the appendicular, but not the axial, skeleton. Jamieson. T.W. et al., Invest. Radiol. 20: 324-9 (1985). Furthermore, IL-1 and TN-α appear to be involved in CIA as in RA. Joosten et al., J. Immunol. 163: 5049.5055, (1999). TNF neutralizing antibodies and separately, TNFR:Fc reduced the symptoms of RA in this model (see Williams et al, PNAS Oct. 1992, 89:9784-9788: Wooley et al., 1993, J. Immunol.151: 6602-6607). Further evidence of the CIA model being predictive of the human condition and response to treatment in RA can be seen, e.g., from the clinical results with ENBREL. The model is described in greater detail in the examples.

In this model for rheumatoid arthritis, type II collagen is purified from bovine articular cartilage (Miller. 1972, Biochemistry 11:4903) and used to immunized mice (Williams et al. 1994, Natl. Acad. Sci. USA 91:2762). Symptoms of arthritis include erythema and/or swelling of the limbs as well as erosions or defects in cartilage and bone as determined by histology. This widely used model is also described, for example, by Holmdahl et al. 1989. APMIS 97:575.

### Articular cartilage explant assay

In this assay, the synthetic and prophylactic potential of the test compounds on the cartilage matrix is described. To this end, proteoglycan (PG) synthesis and breakdown are measured, as well as the release of nitric oxide. Proteoglycans are the second largest component of the organic material in articular cartilage (Kuettner. K.E. et al., Articular Cartilage Biochemistry. Raven Press. New York. USA (1986). p.456: Muir. H., Biochem. Soc. Tran. 11: 613-622 (1983): Hardingham, T.E., Biochem. Soc. Trans. 9:489-497 (1981). Since proteoglycans help determine the physical and chemical properties of cartilage, the decrease in cartilage PGs which occurs during joint degeneration leads to loss of compressive stiffness and elasticity, an increase in hydraulic permeability, increased water content (swelling), and changes in the organization of other extracellular components such as collagens. Thus. PG loss is an early step in the progression of degenerative cartilaginous disorders, one which further perturbs the biomechanical and biochemical stability of the joint. PGs in articular cartilage have been extensively studied because of their likely role in skeletal growth and disease. Mow. V.C., & Ratcliffe, A. Biomaterials 13: 67-97 (1992). Proteoglycan breakdown, which is increased in diseased joints, is measured in the assays described herein by quantitating PGs in the media of explants using the colorimetric DMMB assay. Famdale and Buttle, Biochem. Biophys. Acta 883: 173-177 (1985). Incorporation of ³⁵S-sulfate into proteglycans is used as an indication of proteoglycan synthesis.

The evidence linking IL-1α and degenerative cartilagenous diseases is substantial. For example, high levels of interleukin-1α (IL-1α)(Pelletier JP et al., "Cytokines and inflammation in cartilage degradation" in Osteoarthritic Edition of Rheumatic Disease Clinics of North America. Eds. RW Moskowitz. Philadelphia. W.D. Saunders Company. 1993, p.545-568) and IL-1 receptors (Martel-Pelletier et al., Arthritis Rheum. 35: 530-540 (1992) have been found in diseased joints, and IL-1α induces cartilage matrix breakdown and inhibits synthesis of new matrix molecules. Baragi et al., J. Clin. Invest. 96: 2454-60 (1995): Baragi et al., Osteoarthritis Cartilage 5: 275-82 (1997); Evans et al., J. Leukoc. Biol. 64: 55-61 (1998): Evans et al., J. Rheumatol. 24: 2061-63 (1997): Kang et al., Biochem. Soc. Trans. 25: 533-37 (1997): Kang et al., Osteoarthritis Cartilage 5: 139-43 (1997). Because of the association of IL-1α and IL-1 receptors with diseased tissue, also assayed are the effects of the test compound in the presence of IL-1α. The ability of the test compound to not only have positive effects on cartilage, but also to counteract the catabolic effects of IL-1α, is strong evidence of the protective effect exhibited by the test compound. In addition, such an activity suggests that the test compound could inhibit the degradation which occurs in arthritic conditions, since antagonism of IL-1α function has been shown to reduce the progression of osteoarthritis. Arend, W.P. et al., Ann. Rev. Immunol. 16: 27-55 (1998).

The production of nitric oxide (NO) can be induced in cartilage by catabolic cytokines such as IL-1. Palmer, RMJ et al., Biochem. Biophys. Res. Commun. 193: 398-405 (1993). NO has also been implicated in the joint destruction which occurs in arthritic conditions. Ashok et al., Curr. Opin. Rheum. 10: 263-268 (1998). Unlike normal (undiseased or uninjured) cartilage, cartilage obtained from osteoarthritic joints produces significant amounts of nitric oxide *ex vivo*, even in the absence of added stimuli such as interleukin-1 or lipopolysaccharide. *In vitro*, nitric oxide exerts detrimental effects on chondrocyte function, including inhibition of collagen and proteoglycan synthesis, enhanced apoptosis and inhibition of adhesion to the extracellular matrix. Nitrite concentrations have been shown to be higher in synovial fluid from osteoarthritic patients than in fluid from rheumatoid arthritic patients. Renoux et al., Osteoarthritis Cartilage 4: 175-179 (1996). Furthermore, animal models suggest that inhibition of nitric oxide production reduces progression of arthritis. Pelletier. JP et al., Arthritis Rheum 7:1275-86 (1998); van de Loo et al., Arthritis Rheum. 41:634-46 (1998); Stichtenoth. DO & Frolich J.C. Br. J. Rheumatol. 37: 246-57 (1998). Since NO also has effects on other cells, the presence of NO within the articular joint could increase vasodilation and permeability, potentiate cytokines release by leukocytes, and stimulate angiogenic activity by monocyte-macrophages. Thus, production of NO by cartilage correlates with a diseased state, and since NO appears to play a role in both the erosive and the inflammatory components of joint diseases, a factor which decreases nitric oxide production would likely be beneficial for the treatment of degenerative cartilaginous disorders.

The assay described herein is based on the principle that 2.3-diaminonapthalene (DAN) reacts with nitrite under acidic conditions to form 1-(H)-naphthotriazole, a fluorescent product which can be quantified. As NO is quickly metabolized into nitrite (NO₂⁻¹) and nitrate (NO₃⁻¹), detection of nitrite is one means of detecting (albeit undercounting) the actual NO produced in cartilagenous tissue.

### Mouse Patellae assay

This experiment examines the effects of the test compound on proteoglycan synthesis in the patellae (knee caps) of mice. This assay uses intact cartilage (including the underlying bone) and thus tests factors under conditions which approximate the *in vivo* environment of cartilage. Compounds are either added to patellae *in vitro,* or are injected into knee joints *in vivo* prior to analysis of proteoglycan synthesis in patellae *ex vivo*. As has been shown previously, *in vivo* treated patellae show distinct changes in PG synthesis *ex vivo*. (Van den Berg et al., Rheum. Int. 1: 165-9 (1982): Vershure. P.J. et al., Ann. Rheum. Dis. 53: 455-460 (1994); and Van de Loo et al., Arthrit. Rheum. 38: 164-172 (1995). In this model, the contralateral joint of each animal can be used as a control. The procedure is described in greater detail in the examples.

The following examples are offered for illustrative purposes only, and are not intended to limit the scope of the present invention in any way.

### EXAMPLES

Examples 1-3 describe the treatment of arthritis with an LFA-1 antagonist (anti-murine CD11a antibody, M17) or TNF antagonist (ENBREL: Etanerceptu alone or in combination, in the collagen-induced arthritis (CIA) model. The CIA model is discussed above under Assays/models. Examples 1 and 3 describe treatment of arthritis in two strains of mice, with a combination of anti-murine CD11a antibody (M17) and ENBREL. Example 2 describes treatment with either M17 or ENBREL alone. The preclinical studies for ENBREL used the same animal model.

### EXAMPLE 1- Treatment with an LFA-1 antagonist and a TNF antagonist

DBA-1J mice were immunized with 100 ug bovine collage type II in 100 ul complete Freund's adjuvant (CFA) followed by a second injection of the same collagen in incomplete Freund's adjuvant (IFA) 21 days later. The collagen type II in CFA or IFA was injected intradermally at the base of the tail.

Animals were evaluated every other day. At the onset of arthritis in any of the animals, as noted by swelling of any of the paws, treatment was initiated in all animals placed randomly into the following treatment groups with 12 mice per group.
Group 1. Control Treatment with saline. 100 ul, intraperitoneal route, every day for 14 days, then 3 times per week every other day (Monday. Wednesday and Friday).
Group 2. Anti-murine CD11a monoclonal antibody M17. 150 ug (approx 8 mg/kg) via intraperitoneal route given at onset of disease followed by 3 times per week every other day (Monday. Wednesday and Friday) until the end of the study.
Group 3. ENBREL (human TNF-Fc. 50 ug) intraperitoneal route, given daily at onset of disease for 14 days.
Group 4. Combination of ENBREL and Anti-CDI 1a: Anti-murine CD11a mab M17. 150 ug (approximately 8 mg/kg) via intraperitoneal route given at onset of disease followed by 3 times per week every other day (Monday. Wednesday and Friday) until the end of the experiment. ENBREL. intraperitoneal route, given daily at onset of disease for 14 days.

After the onset of treatment, the mice were evaluated every other day 3 times a week and the severity of paw swelling was subjectively graded for each paw on a scale of 0-4 with 0 = normal. I = minimal. 2 = mild, 3 = moderate, and 4 = severe. A cumulative score was recorded for each animal (potential range 0-16). The animals were terminated 38 days after the initiation of treatment. Radiographs were take on of all four limbs to evaluate for joint lesions and the paws were collected for histopathology. The severity of disease as determined by clinical score for each group (mean +/- standard deviation) was graphed and compared between groups. The clinical scores taken on the last day were corroborated by histological and radiologic analysis of all four paws done at the terminus of the study.

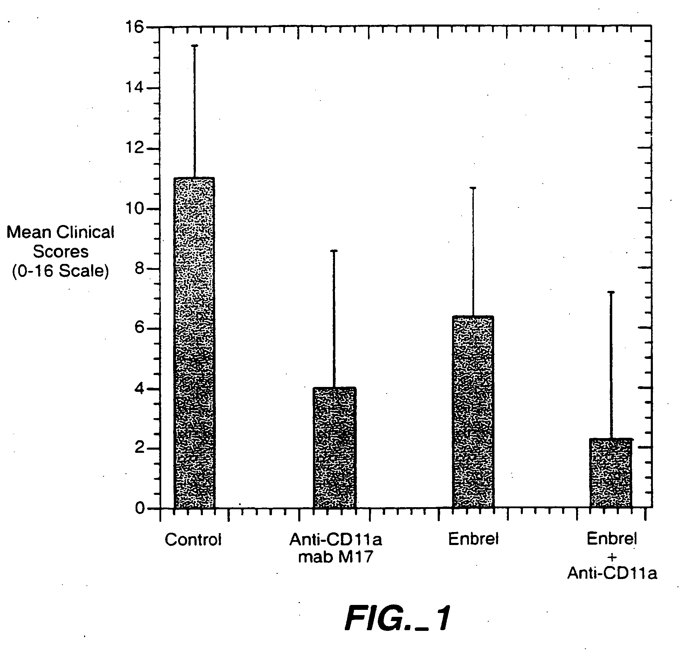

The use of either ENBREL or anti-CD11a antibody reduced the clinical scores compared to the control group (p<0.05) and the combination of anti-CD11a antibody and ENBREL improved the clinical scores compared to ENBREL alone (p<0.05). See Figure 1.

### EXAMPLE 2- Treatment of arthritis with an LFA-1 antagonist or a TNF antagonist

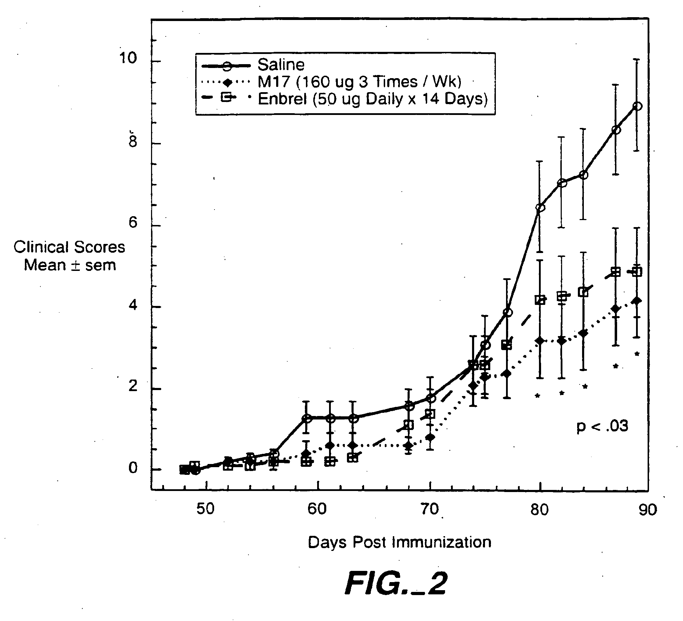

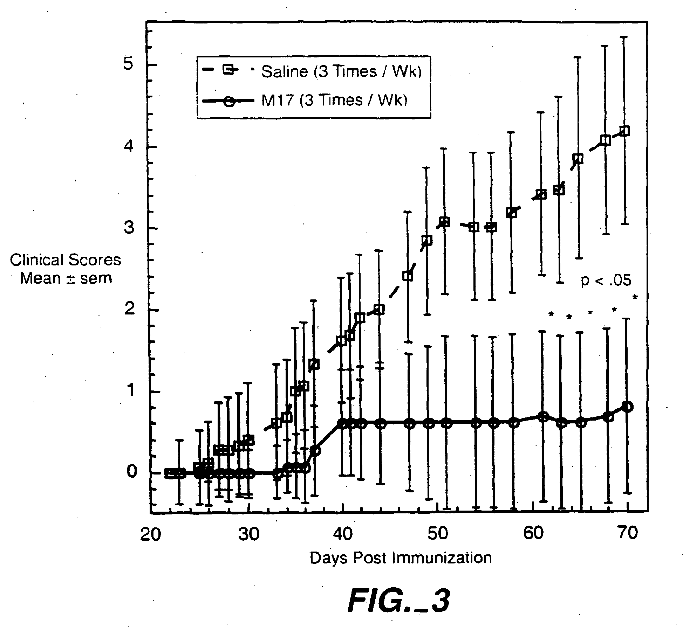

In this example, arthritis was induced in DBA-1LacJ mice (Figure 2) or DBA-1J mice (Figure 3) which were then treated with anti-murine CD11a antibody (M17). ENBREL, or saline as a control. These experiments were performed as described in Example I and in the inset in Figure 2 and Figure 3. Treatment was initiated on day 48 or day 22 post immunization, the day of onset of arthritis in the experiments of Figure 2 and Figure 3. respectively.

The results presented in Figure 2 and Figure 3 show that anti-CD11a antibody or ENBREL alone is effective in treating arthritis as evidenced by the reduction in clinical scores.

### EXAMPLE 3- Treatment of arthritis with an LFA-1 antagonist and a TNF antagonist

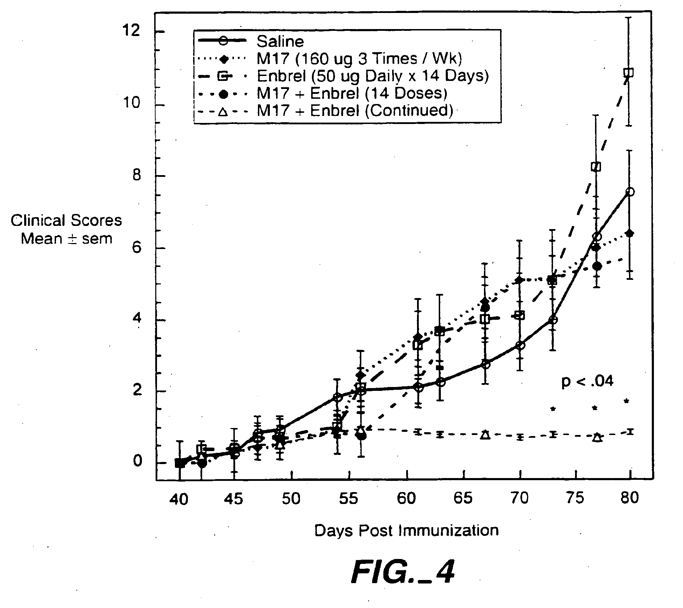

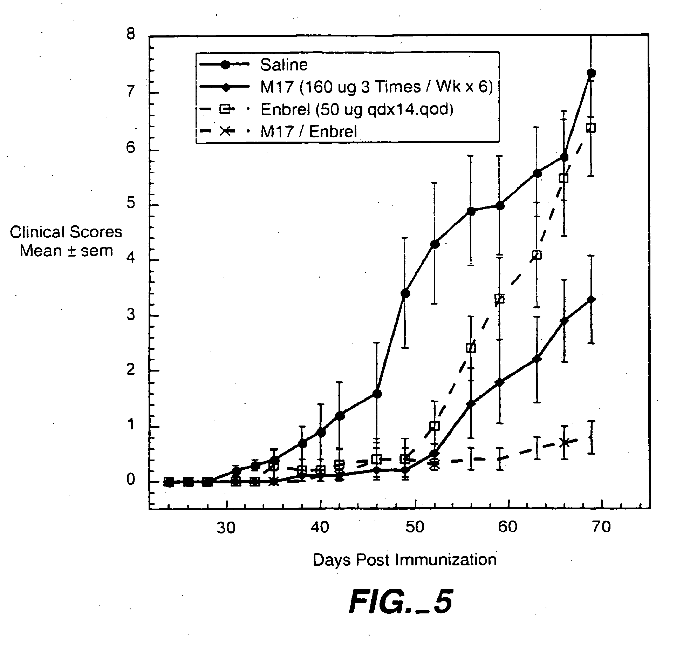

In this example, arthritis was induced in DBA-1LacJ mice (Figure 4) or DBA-1J mice (Figure 5) which were then treated with anti-murine CD11a antibody (M17) alone. ENBREL alone, saline as a control, or a combination of M17 and ENBREL. These experiments were performed as described in Example 1 and in the inset in Figures 4 and 5. In Figure 4. treatment was initiated on day 40 post immunization. M17 was given at 160 ug, three times per week for the duration of the study. For the combination therapy, the mice received 50 ug Enbrel daily up to a total of 14 doses in one experiment, and for the duration of the study in another experiment. In Figure 5. treatment was initiated on day 24 post immunization, the day of onset of arthritis. Enbrel was administered everyday (qd) for 14 days, then every other day (qod). (Monday. Wednesday. Friday) until the end of the experiment.

As is evident from the results shown in Figure 4 and Figure 5, combination therapy with an LFA-1 antagonist and a TNF antagonist had a synergistic effect over treatment with either antagonist alone, resulting in greater reduction in mean clinical scores to almost normal in this animal model.

In Examples 4-6 below, a test compound refers to an LFA-1 antagonist (e.g.. anti-CD11a antibody) or a TNF antagonist (e.g., ENBREL). The volumes, concentrations and time points are exemplary and can be varied as will be familiar to one of skill in the art.

### EXAMPLE 4 - Articular cartilage explant assay

This assay, discussed above under Assays/Models, examines both the synthetic and prophylactic potential of a test compound on the cartilage matrix. This potential is determined both by stimulation of matrix synthesis and inhibition of matrix breakdown, as determined by: (1) PG synthesis in the articular matrix; (2) Inhibition of PG release; (3) Inhibition of IL-1α induced breakdown: and (4) Inhibition of nitric oxide.

### Articular cartilage explants

The metacarpophalangeal joint of 4-6 month old female pigs is aseptically opened, and articular cartilage is dissected free of the underlying bone. The cartilage is minced, washed and cultured in bulk for at least 24 hours in a humidified atmosphere of 95% air and 5% CO₂ in serum free low glucose 50:50 DMEM:F12 media with 0.1% BSA. 100U/ml penicillin/streptomycin (Gibco). 2mM L-glutamine, 1x GHT. 0.1mM MEM Sodium Pyruvate (Gibco), 20 µg/ml Gentamicin (Gibco). 1.25mg/L Amphotericin B (Sigma). 5µg/mL Vitamin E and 10µg/mL transferrin. Approximately 50mg of articular cartilage is aliquoted into Micronics tubes and incubated for at least 24 hours in above media before being changed into media without Vitamin E and transferrin. Test proteins are then added. Media is harvested and changed at various time points (e.g., 0. 24, 48. 72h).

### Measurement of proteoglycans:

DMMB is a dye that undergoes metachromasia (a change in color, in this case from blue to purple) upon binding to sulfated glycosaminoglycans (GAG), the side-chains of proteoglycans. The addition of sulfated proteoglycans to DMMB causes a decrease in the peak values at 590 and 660 nm with an increase in absorbance at 530 nm. The amount of proteoglycans in media is determined by adding DMMB dye in a 96 well plate format, and the change in color is quantitated using a spectrophotometer (Spectramax 250). The DMMB assay is a well-accepted method to measure the amount of proteoglycans in cartilage cultures. For this assay, a standard curve is prepared using chondroitin sulfate ranging from 0.0 to 5.0 µg. The procedure has been adapted from the colorimetric assay described in Farndale and Buttle. Biochem. Biophys, Acta 883: 173-177 (1986).

### Measurement of proteoglycan synthesis in articular cartilage explants

After the media change at 48 hr. ³⁵S-sulfate (to a final concentration of 10 µCi/ml) is added to the cartilage explants. After an overnight incubation at 37°C. media is saved for measurements of nitric oxide or proteoglycan content. Cartilage pieces are washed two times using explant media. 900 µl digestion buffer containing 10 mM EDTA, 0.1 M Sodium phosphate and 1 mg/ml proteinase K (Gibco BRL) is added to each tube and incubated overnight in a 50°C water bath. 600 µL of the digest is mixed with 600 µL of 10% w/v cetylpyridinium chloride (Sigma). Samples are spun at 1000 x g for 15 min. The supernatant is removed, and 500 µL formic acid (Sigma) is added to the samples to dissolve the precipitate. Solubilized pellets are transferred to scintillation vials containing 10 ml scintillation fluid (ICN), and samples are read in a scintillation counter.

### Measurement of nitric oxide (NO)

10 µL of 0.05 mg/ml 2,3-diaminonapthalene (DAN) in 0.62M HCI is added to 100 µL media from cartilage explants. Samples are mixed and incubated at room temperature for 10-20 minutes. The reaction is terminated with 5 µL of 2.8 M NaOH. The fluorescent product, 2,3-diaminonaphthotriazole, is measured using a Cytoflor fluorescent plate reader with excitation at 360 nm and emission read at 450 nm.

### EXAMPLE 5 -Mouse Patellae Assay

This assay determines the in *vivo* effect of an LFA-1 antagonist and a TNF antagonist (e.g., anti-CD11a antibody and ENBREL) on proteoglycan synthesis in the patellae of mice. The patella is a very useful model because it permits the evaluation of the effects of a test compound on cartilage which has not been removed from the underlying bone. Moreover, the evaluation of localized ambular *in vivo* injections offers virtually ideal experimental controls, since each animal has two patellae in separate and distinct regions of their body. The procedure herein is adapted from the one outlined in Van den Berg *et al., Rheum. Im*. 1: 165-9 (1982): Vershure P.J. *et al., Ann. Rheum. Dis*. 53: 455-460 (1994); and Van de Loo *et al., Arthit. Rheum*. 38: 164-172 (1995). This assay is discussed above under Assays/Models.

In the *ex vivo* treatment group, the patellae of mice are carefully removed and incubated overnight in media with one of the following; no additional factors (e.g., saline control); IL-1 e.g., at 100 ng/ml): anti-CD11a antibody or ENBREL: IL-11 and anti-CD11a antibody or IL-1 and ENBREL: anti-CD11a antibody and ENBREL in combination: to look for the ability of the test compound(s) to inhibit the effects of IL-1 During the last 3 hours of the incubation, 30 Ci/ml ³⁵S-sulfur is added for 3 hours in a tissue culture incubator followed by three washings with PBS. Samples are then fixed overnight in 10% formalin followed by decaling in 5% formic acid for at least 5 hours. The cartilage is dissected away from the underlying bone and placed in 500 I of solvent and incubated at 60°C for 1.5 hours, 10 ml of HIONIC-fluor is added to each tube and mixed thoroughly. The solution is transferred into scintillation vials and ³⁵S uptake as a measure of PG synthesis is then determined on a scintillation counter.

In the *in vivo* treatment group, animals are separated into two subgroups and injected (e.g., into knee joints) with the test compounds individually or in combination (e.g., anti-CD11a antibody and ENBREL) into one knee. The dosage and dosing regimen is varied to define the optimum conditions for treatment. The patellae are then harvested and assayed as described above.

## Claims

1. Use of an LFA-1 antagonist and a TNF-α antagonist in the preparation of a medicament for treating rheumatoid arthritis, wherein the LFA-1 antagonist is an anti-CD11a antibody, and the TNF-α antagonist is an immunoadhesin comprising at least a TNF-α binding portion of the p75 TNF-α receptor.

2. Use according to claim 1, wherein the medicament further comprises methotrexate.

3. Use according to claim 1 or claim 2, wherein said medicament comprises the LFA-1 antagonist and the TNF-α antagonist for sequential administration.

4. Use according to claim 3, wherein said medicament comprises the LFA-1 antagonist and the TNF-α antagonist for concurrent administration.

5. Use according to any one of claims 1 to 4, wherein the anti-CD11a antibody is a non T-cell depleting antibody.

6. Use according to any one of claims 1 to 5, wherein the TNF-α antagonist is an immunoadhesin consisting of the extracellular ligand binding portion of human p75 tumor necrosis factor receptor linked to the hinge region, CH2 domain, and CH3 domain of human IgG1.

7. A composition comprising an LFA-1 antagonist and a TNF-α antagonist, wherein the LFA-1 antagonist is an anti-CD11a antibody, and the TNF-α antagonist is an immunoadhesin comprising at least a TNF-α binding portion of the p75 TNF-α receptor.

8. A composition according to claim 7, wherein said medicament comprises the LFA-1 antagonist and the TNF-α antagonist for sequential administration.

9. A composition according to claim 7, wherein said medicament comprises the LFA-1 antagonist and the TNF-α antagonist for concurrent administration.

10. A composition according to claim 7, wherein the anti-CD11a antibody is a non T-cell depleting antibody.

11. A composition according to any one of claims 7 to 10, wherein the TNF-α antagonist is an immunoadhesin consisting of the extracellular ligand binding portion of human p75 tumor necrosis factor receptor linked to the hinge region, CH2 domain, and CH3 domain of human IgG1.

12. A therapeutic kit comprising an LFA-1 antagonist and a TNF-α antagonist, and a carrier, excipient and/or stabilizer, in suitable packaging, wherein the antagonists in said first and second containers are for sequential or concurrent administration, wherein the LFA-1 antagonist is an anti-CD11a antibody, and the TNF-α antagonist is an immunoadhesin comprising at least a TNF-α binding portion of the p75 TNF-α receptor.

13. An LFA-1 antagonist and a TNF-α antagonist for use in a method of medical treatment, wherein the LFA-1 antagonist is an anti-CD11a antibody, and the TNF-α antagonist is an immunoadhesin comprising at least a TNF-α binding portion of the p75 TNF-α receptor.

## Patentansprüche

1. Verwendung eines LFA-1-Antagonisten und eines TNF-α-Antagonisten zur Herstellung eines Medikaments zur Behandlung von rheumatoider Arthritis, worin der LFA-1-Antagonist ein Anti-CD11a-Antikörper und der TNF-α-Antagonist ein Immunoadhäsin ist, das zumindest einen TNF-α-Bindungsabschnitt des p75-TNF-α-Rezeptors umfasst.

2. Verwendung nach Anspruch 1, worin das Medikament weiters Methotrexat umfasst.

3. Verwendung nach Anspruch 1 oder 2, worin das Medikament den LFA-1-Antagonisten und den TNF-α-Antagonisten zur sequenziellen Verabreichung umfasst.

4. Verwendung nach Anspruch 3, worin das Medikament den LFA-1-Antagonisten und den TNF-α-Antagonisten zur gleichzeitigen Verabreichung umfasst.

5. Verwendung nach einem der Ansprüche 1 bis 4, worin der Anti-CD11a-Anti-körper einer ist, der keine T-Zellen abreichert.

6. Verwendung nach einem der Ansprüche 1 bis 5, worin der TNF-α-Antagonist ein Immunoadhäsin ist, das aus dem extrazellulären Ligandenbindungsabschnitt des an die Gelenksregion, CH2-Domäne und CH3-Domäne von humanem IgG1 gebundenen humanen p75-Tumornekrosefaktorrezeptors besteht.

7. Zusammensetzung, umfassend einen LFA-1-Antagonisten und einen TNF-α-Antagonisten, worin der LFA-1-Antagonist ein Anti-CD11a-Antikörper und der TNF-α-Antagonist ein Immunoadhäsin ist, das zumindest einen TNF-α-Bindungsabschnitt des p75-TNF-α-Rezeptors umfasst.

8. Zusammensetzung nach Anspruch 7, worin das Medikament den LFA-1-Antagonisten und den TNF-α-Antagonisten zur sequenziellen Verabreichung umfasst.

9. Zusammensetzung nach Anspruch 7, worin das Medikament den LFA-1-Antagonisten und den TNF-α-Antagonisten zur gleichzeitigen Verabreichung umfasst.

10. Zusammensetzung nach Anspruch 7, worin der Anti-CD11a-Antikörper einer ist, der keine T-Zellen abreichert.

11. Zusammensetzung nach einem der Ansprüche 7 bis 10, worin der TNF-α-Antagonist ein Immunoadhäsin ist, das aus dem extrazellulären Ligandenbindungsabschnitt des an die Gelenksregion, CH2-Domäne und CH3-Domäne von humanem IgG1 gebundenen humanen p75-Tumornekrosefaktorrezeptors besteht.

12. Therapeutisches Set, umfassend einen LFA-1-Antagonisten und einen TNF-α-Antagonisten sowie einen Träger, einen Arzneimittelträger und/oder Stabilisator in geeigneter Verpackung, worin die Antagonisten in dem ersten und zweiten Behälter der sequenziellen oder gleichzeitigen Verabreichung dienen, worin der LFA-1-Antagonist ein Anti-CD11a-Antikörper und der TNF-α-Antagonist ein Immunoadhäsin ist, das zumindest einen TNF-α-Bindungsabschnitt des p75-TNF-α-Rezeptors umfasst.

13. LFA-1-Antagonist und ein TNF-α-Antagonist zur Verwendung in einem Verfahren der medizinischen Behandlung, worin der LFA-1-Antagonist ein Anti-CD11a-Antikörper und der TNF-α-Antagonist ein Immunoadhäsin ist, das zumindest einen TNF-α-Bindungsabschnitt des p75-TNF-α-Rezeptors umfasst.

## Revendications

1. Utilisation d'un antagoniste de LFA-1 et d'un antagoniste du TNF-α dans la préparation d'un médicament destiné au traitement de la polyarthriterhumatoïde, dans laquelle l'antagoniste de LFA-1 est un anticorps anti-CD11a et l'antagoniste du TNF-α est une immunoadhésine comprenant au moins une portion de liaison au TNF-α du récepteur de TNF-α p75.

2. Utilisation suivant la revendication 1, dans laquelle le médicament comprend en outre du méthotrexate.

3. Utilisation suivant la revendication 1 ou la revendication 2, dans laquelle ledit médicament comprend l'antagoniste de LFA-1 et l'antagoniste du TNF-α pour une administration séquentielle.

4. Utilisation suivant la revendication 3, dans laquelle ledit médicament comprend l'antagoniste de LFA-1 et l'antagoniste du TNF-α pour une administration conjointe.

5. Utilisation suivant l'une quelconque des revendications 1 à 4, dans laquelle l'anticorps anti-CD11a est un anticorps à non-déplétion lymphocytaire T.

6. Utilisation suivant l'une quelconque des revendications 1 à 5, dans laquelle l'antagoniste du TNF-α est une immunoadhésine consistant en la portion de liaison de ligand extracellulaire du récepteur de facteur de nécrose de tumeur p75 humain liée à la région de charnière, au domaine CH2 et au domaine CH3 d'une IgG1 humaine.

7. Composition comprenant un antagoniste de LFA-1 et un antagoniste du TNF-α, dans laquelle l'antagoniste de LFA-1 est un anticorps anti-CD11a et l'antagoniste du TNF-α est une immunoadhésine comprenant au moins une portion de liaison au TNF-α du récepteur de TNF-α p75.

8. Composition suivant la revendication 7, dans laquelle ledit médicament comprend l'antagoniste de LFA-1 et l'antagoniste du TNF-α pour une administration séquentielle.

9. Composition suivant la revendication 7, dans laquelle ledit médicament comprend l'antagoniste de LFA-1 et l'antagoniste du TNF-α pour une administration conjointe.

10. Composition suivant la revendication 7, dans laquelle l'anticorps anti-CD11a est un anticorps à non-déplétion lymphocytaire T.

11. Composition suivant l'une quelconque des revendications 7 à 10, dans laquelle l'antagoniste du TNF-α est une immunoadhésine consistant en la portion de liaison de ligand extracellulaire du récepteur du facteur de nécrose de tumeur p75 humain liée à la région de charnière, au domaine CH2 et au domaine CH3 d'une IgG1 humaine.

12. Kit thérapeutique comprenant un antagoniste de LFA-1 et un antagoniste du TNF-α, et un support, excipient et/ou stabilisant, dans un emballage convenable, dans lequel les antagonistes dans lesdits premier et second récipients sont destinés à une administration séquentielle ou conjointe, et dans lequel l'antagoniste de LFA-1 est un anticorps anti-CD11a et l'antagoniste du TNF-α est une immunoadhésine comprenant au moins une portion de liaison de TNF-α et du récepteur de TNF-α p75.

13. Antagoniste de LFA-1 et antagoniste du TNF-α destinés à être utilisés dans une méthode de traitement médical, où l'antagoniste de LFA-1 est un anticorps anti-CD11a et l'antagoniste du TNF-α est une immunoadhésine comprenant au moins une portion de liaison de TNF-α du récepteur de TNF-α p75.
